# EUROPEAN PATENT APPLICATION

(11) **EP 4 364 722 A1**
(43) Date of publication of application: **08.05.2024**
(21) Application number: 22864985.1
(22) Date of filing: 29.08.2022
(51) Int. Cl.: A61K 9/00, A61K 9/08, A61K 9/107, A61K 31/7042, A61K 31/7034, A61K 31/7048, A61P 27/02

(54) **PHARMACEUTICAL COMPOSITION FOR PREVENTING OR TREATING DIABETIC EYE DISEASE COMPRISING SGLT-2 INHIBITOR**

(30) Priority: 02.09.2021 KR 20210117133; 26.08.2022 KR 20220107682
(71) Applicant: Daewoong Therapeutics Inc., Suwon-si, Gyeonggi-do 16226 (KR)
(72) Inventor: PARK, Sang Han, Seoul 07694 (KR); KANG, Bok Ki, Seongnam-si Gyeonggi-do 13539 (KR); KIM, Dong Hwan, Suwon-si Gyeonggi-do 16336 (KR); KANG, Min Hyeong, Yongin-si Gyeonggi-do 17066 (KR)
(74) Representative: Stolmár & Partner Patentanwälte PartG mbB
(86) International application number: PCT/KR2022/012895
(87) International publication number: WO 2023/033483

(57) **Abstract**

The present invention relates to a pharmaceutical composition for preventing or treating diabetic eye disease, comprising sodium-glucose cotransporter-2 (SGLT-2) inhibitor, and a method for preventing or treating diabetic eye disease by using same.

## Description

### TECHNICAL FIELD

The present invention relates to a pharmaceutical composition for preventing or treating a diabetic eye disease, comprising a sodium-glucose cotransporter-2 (SGLT-2) inhibitor, and a method of preventing or treating a diabetic eye disease using the same.

### BACKGROUND ART

Due to Western diet patterns and the like, the rate of blindness caused by diabetes and subsequent diabetic eye diseases is gradually increasing, and the burden of medical expenses is increasing accordingly.

As one of the diabetic eye diseases, diabetic retinopathy (DR) is the most common microvascular complex, and is the main cause of impaired vision in diabetic patients and the working-age population. Although new blood vessels cause serious vision damage in the later stages of diabetic retinopathy as diabetic retinopathy progresses, macular edema caused by blood vessel leakage may occur at any stage of diabetic retinopathy and may impair vision.

In particular, diabetic retinopathy often appears 10 to 15 years after the onset of diabetes even when diabetes is well controlled and blood sugar levels are maintained in a normal range. Diabetic retinopathy is the biggest cause of blindness in diabetic patients, and is also considered one of the world's top three diseases that pose a risk of blindness.

Such a diabetic eye disease has a very high risk of blindness. Therefore, although it has a high therapeutic effect, a therapeutic agent that is administered to the eyeballs causes discomfort to patients due to the nature of administration. Therefore, there is a continuous need for a therapeutic agent that takes into account the patient's convenience in taking medication.

### DISCLOSURE

### TECHNICAL PROBLEM

The technical problem to be solved by the present invention is to provide a pharmaceutical composition for preventing or treating a diabetic eye disease, comprising an SGLT-2 inhibitor or a pharmaceutically acceptable salt thereof.

Also, the technical problem to be solved by the present invention is to provide a method of preventing or treating a diabetic eye disease, comprising: administering the pharmaceutical composition to a subject.

### TECHNICAL SOLUTION

According to an aspect of the present invention relates to pharmaceutical composition for preventing or treating a diabetic eye disease, comprising an SGLT-2 inhibitor or a pharmaceutically acceptable salt thereof.

In the present invention, the term "sodium-glucose cotransporter-2 (SGLT-2) inhibitor" refers to a drug that inhibits glucose reabsorption by inhibiting SGLT-2 in the proximal tubules of the kidney.

In one embodiment, the SGLT-2 inhibitor may be at least one from the group consisting of ertugliflozin, ipragliflozin, dapagliflozin, empagliflozin, tofogliflozin, and pharmaceutically acceptable salts thereof, but the present invention is not limited thereto.

In one embodiment, the ertugliflozin may also be represented by the following Formula 1.

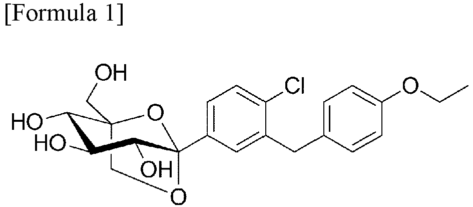

The ipragliflozin may be represented by the following Formula 2.

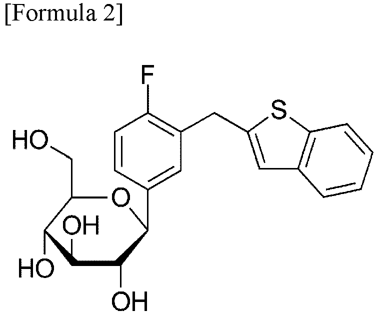

The dapagliflozin may be represented by the following Formula 3.

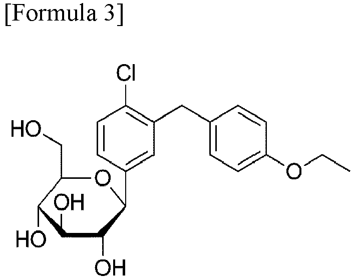

The empagliflozin may be represented by the following Formula 4.

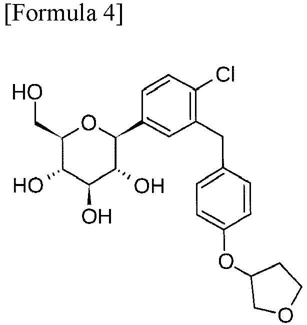

The tofogliflozin may be represented by the following Formula 5.

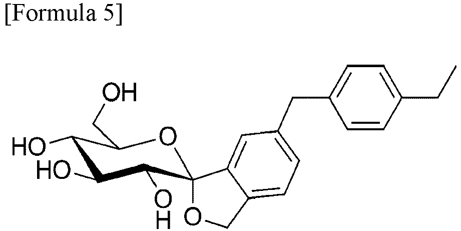

In the present invention, the term "diabetic eye disease" refers to all types of eye diseases caused by diabetes. In general, diabetic eye diseases develop due to waste accumulation or bleeding as the blood vessels that supply nutrients to the retina located at the back of the eye become weak. In one embodiment, the diabetic eye disease may include one or more selected from the group consisting of diabetic retinopathy, macular edema, cataracts, glaucoma, and paralytic strabismus, but the present invention is not limited thereto. In this case, the diabetic eye disease may include all types of eye diseases that occur in diabetic patients or develop as one of the complications caused by diabetes.

Diabetes is a metabolic disease that causes lesions in microvessels. When hyperglycemia persists due to diabetes, the capillary vessels in the retina of the eye are destroyed and new blood vessels are created. The blood vessels thus created bleed easily, causing the retina to detach. As a result, diabetic retinopathy is a disease in which the function of the optic nerve is impaired due to the poor supply of nutrients to the eyes. The diabetic retinopathy comprises both non-proliferative retinopathy and proliferative retinopathy.

The "macular edema" is a disease in which edema occurs in the macula present in the center of the retina. When the blood vessels in the macula are blocked due to diabetes, new blood vessels are formed. In this case, fluids or blood leaks from these weak blood vessels and accumulates, thereby causing macular edema.

The "glaucoma" is a disease in which abnormalities occur in the optic nerve due to various risk factors, such as an increase in constant pressure (intraocular pressure) maintained inside the eyeballs, and when left untreated, the field of view gradually becomes narrower, leading to blindness. Glaucoma is known to occur three times more often in diabetic patients than in normal people. It was reported that intraocular pressure, which is the most important factor in the onset of glaucoma, increases to 21 mmHg or more in diabetic patients, which is twice as high as in normal people.

The "cataract" is a disease in which the eye lens becomes cloudy and loses transparency. In the case of diabetic patients, the probability of developing a cataract is approximately 5 times higher than that of normal people, and approximately 13% of all patients show signs of cataracts. The onset of cataracts is also known to occur at a relatively early age in diabetic patients.

The "paralytic strabismus" refers to strabismus that develops due to problems in a process of transmitting electrical signals from the brain to the muscles that move the eyeballs. 36 to 42% of patients with paralytic strabismus are diabetic patients, and the frequency of paralytic strabismus is six times higher in diabetic patients than in non-diabetic patients. In the case of diabetic patients, various abnormal metabolic processes are accelerated due to high blood sugar. During this process, harmful substances such as oxygen radicals, advanced glycation end products, and the like accumulate, thereby causing nerve and blood vessel damage. Due to this blood vessel damage, the blood supply to the nerves that control the muscles that move the eyeballs may be diminished, which results in insufficient nutritional supply, and nerve damage may be accelerated due to hypoxia, which leads to paralytic strabismus due to diabetes.

The composition of the present invention may be administered parenterally, and in one embodiment may be administered to the eyeballs. In particular, the composition may be in the form of eye drops for eye drop administration.

Also, in one embodiment, the composition may be administered once a day for eye drop administration, but the present invention is not limited thereto.

In one embodiment of the present invention, a composition comprising an SGLT-2 inhibitor was administered as eye drops to the eye, and it was confirmed that the composition has an excellent therapeutic effect on diabetic eye diseases. Particularly, in contrast to conventional therapies for eye diseases administered via intravitreal injections, the composition of the present invention can be conveniently administered to the eye as eye drops, greatly enhancing medication convenience. As a result, the composition of the present invention can be effectively applied to the treatment of eye diseases.

In the present invention, the term "prevention" refers to any action in which the composition of the present invention inhibits the onset of diabetic eye diseases.

In the present invention, the term "treatment" refers to any action in which the composition of the present invention improves or benefits the symptoms of diabetic eye diseases.

The pharmaceutical composition of the present invention may be applied in a pharmaceutically effective amount, and the term "pharmaceutically effective amount" refers to an amount sufficient to treat a disease with a reasonable benefit/risk ratio applicable to medical treatment, and a level of the effective dose may be determined by factors comprising the sex and age of a patient, the type and severity of a disease, the activity of a drug, the sensitivity to the drug, the time of administration, the route of administration, and the excretion rate, the duration of treatment, concurrently used drugs, and other factors well known in the medical field.

Another aspect of the present invention relates to a method of preventing or treating a diabetic eye disease, which comprises administering the pharmaceutical composition to a subj ect.

In the present invention, the term "subject" comprises animals or humans with diabetic eye diseases whose symptoms can be improved by administration of the pharmaceutical composition according to the present invention. The "diabetic eye disease" is as described above.

The diabetic eye disease may be effectively prevented and treated by administering the therapeutic composition according to the present invention to a subject.

In the present invention, the term "administration" refers to a process of introducing a certain substance into a human or animal using any suitable method, and the route of administration of the therapeutic composition according to the present invention is oral or parenteral via any general route as long as it can reach the target tissue. Also, the therapeutic composition according to the present invention may be administered by any device that can deliver an active ingredient to target cells.

The preferred dose of the pharmaceutical composition according to the present invention varies depending on the patient's condition and weight, the severity of a disease, the type of a drug, the route of administration route, and the administration period, but may be appropriately selected by those skilled in the art.

### ADVANTAGEOUS EFFECTS OF INVENTION

A pharmaceutical composition comprising an SGLT-2 inhibitor or its pharmaceutically acceptable salt as described in the present invention can exhibit excellent therapeutic effects on diabetic eye diseases.

In particular, it offers the advantage of convenient administration as an eye drop once a day to the eye, unlike conventional therapies administered via intravitreal injections, greatly enhancing medication convenience and preventing damage to ocular tissues and cells caused by injections.

The effects of the present invention are not limited to those mentioned above, and should be understood to encompass all effects that can be inferred from the detailed description or the claims of the invention.

### BRIEF DESCRIPTION OF THE DRAWINGS

FIG. 1 shows the results of measuring fluorescence intensity on 21st day after administration of the composition of the present invention.
FIG. 2 shows images of the fundus photographed on 21st day after administration of the composition of the present invention.

### DETAILED DESCRIPTION OF EXEMPLARY EMBODIMENTS

Hereinafter, the present invention will be described in detail with reference to exemplary embodiments thereof. However, it should be understood that the following exemplary embodiments are merely illustrative of the present invention and are not intended to limit the present invention.

### Preparation Example 1: Preparation of eye drop formulation comprising SGLT-2 inhibitor

A measured amount of injection-grade water, a solubilizer, and an osmotic pressure regulator were added to a glass vial. Subsequently, the mixture was heated and stirred at a temperature of 60°C or higher until it was completely dissolved. The active ingredient was then weighed and gradually added to the glass vial, completely dissolving it. Following this, sodium hydroxide and sodium phosphate dibasic were used to adjust the pH to the appropriate range, around a pH level of 7, and the remaining measured amount of water for injection was added. The resulting clear solution was aseptically filtered through a 0.22 µm filter and filled.

One of the following SGLT-2 inhibitors was used as the active ingredient, and the composition for each concentration is as summarized below.

### Example 1: 2.0% Ertugliflozin-containing eye drops

| Purpose of use | Ingredient | Concentration (mg/ml) | Content (w/v%) |
|---|---|---|---|
| Active ingredient | Ertugliflozin | 20 | 2.0 |
| Solubilizer | Kolliphor ELP | 70 | 7.0 |
| Solubilizer | Polyoxyl 40 stearate | 20 | 2.0 |
| Solubilizer | Tween 80 | 5 | 0.5 |
| Solubilizer | Vitamin E TPGS1000 (Tocopherol | 20 | 2.0 |
| | polyethylene glycerol 1000 succinate) | | |
| Osmotic pressure regulator | Glycerin | 0.12 | 0.012 |
| pH regulator | Anhydrous sodium dihydrogen phosphate | 0.0198 | 0.002 |
| pH regulator | Sodium hydroxide | 70 | 7.0 |
| Solvent | Water for injection | q.s. to 100 | q.s. to 100 |

### Example 2: 1.0% Ipragliflozin-containing eye drops

| Purpose of use | Ingredient | Concentration (mg/ml) | Content (w/v%) |
|---|---|---|---|
| Active ingredient | Ipragliflozin | 20 | 2.0 |
| Solubilizer | Polyoxyl 40 stearate | 70 | 7.0 |
| Solubilizer | Tween 80 | 20 | 2.0 |
| Solubilizer | Vitamin E TPGS1000 (Tocopherol polyethylene glycerol 1000 succinate) | 5 | 0.5 |
| Osmotic pressure regulator | Glycerin | 20 | 2.0 |
| pH regulator | Anhydrous sodium dihydrogen phosphate | 0.12 | 0.012 |
| pH regulator | Sodium hydroxide | 0.0198 | 0.002 |
| Solvent | Water for injection | q.s. to 100 | q.s. to 100 |

### Example 3: 2.0% Dapagliflozin-containing eye drops

| Purpose of use | Ingredient | Concentration (mg/ml) | Content (w/v%) |
|---|---|---|---|
| Active ingredient | Dapagliflozin | 20 | 2.0 |
| Solubilizer | Polyoxyl 40 stearate | 70 | 7.0 |
| Solubilizer | Tween 80 | 20 | 2.0 |
| Solubilizer | Vitamin E TPGS1000 (Tocopherol polyethylene glycerol 1000 succinate) | 5 | 0.5 |
| Osmotic pressure regulator | Glycerin | 20 | 2.0 |
| pH regulator | Anhydrous sodium dihydrogen phosphate | 0.12 | 0.012 |
| pH regulator | Sodium hydroxide | 0.0198 | 0.002 |
| Solvent | Water for injection | q.s. to 100 | q.s. to 100 |

### Example 4: 1.0% Empagliflozin-containing eye drops

| Purpose of use | Ingredient | Concentration (mg/ml) | Content (w/v%) |
|---|---|---|---|
| Active ingredient | Empagliflozin | 10 | 1.0 |
| Solubilizer | Polyoxyl 40 stearate | 70 | 7.0 |
| Solubilizer | Tween 80 | 20 | 2.0 |
| Solubilizer | Vitamin E TPGS1000 (Tocopherol polyethylene glycerol 1000 succinate) | 5 | 0.5 |
| Osmotic pressure regulator | Glycerin | 20 | 2.0 |
| pH regulator | Anhydrous sodium dihydrogen phosphate | 0.12 | 0.012 |
| pH regulator | Sodium hydroxide | 0.0198 | 0.002 |
| Solvent | Water for injection | q.s. to 100 | q.s. to 100 |

### Example 5: 2.0% Tofogliflozin-containing eye drops

| Purpose of use | Ingredient | Concentration (mg/ml) | Content (w/v%) |
|---|---|---|---|
| Active ingredient | Tofogliflozin | 20 | 2.0 |
| Solubilizer | Polyoxyl 40 stearate | 70 | 7.0 |
| Solubilizer | Tween 80 | 20 | 2.0 |
| Solubilizer | Vitamin E TPGS1000 (Tocopherol polyethylene glycerol 1000 succinate) | 5 | 0.5 |
| Osmotic pressure regulator | Glycerin | 20 | 2.0 |
| pH regulator | Anhydrous sodium dihydrogen phosphate | 0.12 | 0.012 |
| pH regulator | Sodium hydroxide | 0.0198 | 0.002 |
| Solvent | Water for injection | q.s. to 100 | q.s. to 100 |

### Comparative Example 1: Vehicle

A combination of common excipients excluding the active ingredients of each type of eye drop was prepared and administered as Comparative Example 1.

| Purpose of use | Ingredient | Concentration (mg/ml) | Content (w/v%) |
|---|---|---|---|
| Solubilizer | Polyoxyl 40 stearate | 70 | 7.0 |
| Solubilizer | Tween 80 | 20 | 2.0 |
| Solubilizer | Vitamin E TPGS1000 (Tocopherol polyethylene glycerol 1000 succinate) | 5 | 0.5 |
| Osmotic pressure regulator | Glycerin | 20 | 2.0 |
| pH regulator | Anhydrous sodium dihydrogen phosphate | 0.12 | 0.012 |
| pH regulator | Sodium hydroxide | 0.0198 | 0.002 |
| Solvent | Water for injection | q.s. to 100 | q.s. to 100 |

### Comparative Example 2: Ranibizumab

LUCENTIS^{®} was purchased and administered as a positive control.

### Experimental Example 1: Confirmation of pharmacological effects on diabetic retinopathy

### 1-1. Production of diabetic retinopathy animal model

Brown Norway rats (BN/SsN Sic) were used. During the 14-day acclimation period after acquisition, general symptoms were observed to confirm the rats' physical conditions, and 36 healthy males were used in this experiment.

Rats were raised under set conditions of a temperature of 23 ± 3°C, a relative humidity of 55 ± 15%, a ventilation frequency of 10 to 20 times/hr, a lighting time of 12 hours, and a luminance of 150 to 300 Lux. During the breeding period, environmental conditions such as the temperature/ humidity, ventilation frequency, and luminance of the animal breeding room were measured regularly. During the acclimation, administration, and observation periods, the rats were raised in polycarbonate breeding boxes (W 170 mm x L 235 mm x H 125 mm) for rodents with no more than 3 animals/box, and the breeding boxes, bedding, and water bottles were changed at least once a week.

Streptozotocin (STZ) was administered to animals determined to be healthy during the acclimation period, and blood sugar levels were measured on day 7 of administration. Only the animals with a measured value of 300 mg/dL or higher were selected and randomly divided so that the average blood sugar levels in each group could be distributed as uniformly as possible according to the ranked blood sugar levels. Streptozotocin was intravenously administered once at a dose of 60 mg/kg on the day of induction.

### 1-2. Configuration of test group and dose settings

The configuration of the test group and the dosage settings of the test materials are as shown in Table 1 below. G1 is a normal control in which diabetic retinopathy was not induced, G2 is a control in which diabetic retinopathy was induced and to which the vehicle of Comparative Example 1 was administered, and G3 and G4 are groups to which ranibizumab of Comparative Example 2 was administered as a positive control material, and G5 to G9 are groups to which the SGLT-2 inhibitors according to Examples 1 to 5 were administered, respectively.

**[Table 1]**

| Grou p | Sex | Numbe r of animal s | Administrati on material | Inductio n | Route of administratio n | Dose of active ingredient (µg/eye) | Injectio n volume (µL/eye) | Administrat ion frequency |
|---|---|---|---|---|---|---|---|---|
| G1 | Mal e | 4 | Saline | N | - | - | - | - |
| G2 | Mal e | 4 | Comparative Example 1 | Y | Eye drops | - | 10 | Once a day |
| G3 | Mal e | 4 | Comparative Example 2 | Y | Intravitreal | 50 | 5 | Once |
| G4 | Mal e | 4 | Comparative Example 2 | Y | Intravitreal | 10 | 1 | Once |
| G5 | Mal e | 4 | Example 1 | Y | Eye drops | 200 | 10 | Once a day |
| G6 | Mal e | 4 | Example 2 | Y | Eye drops | 100 | 10 | Once a day |
| G7 | Mal e | 4 | Example 3 | Y | Eye drops | 200 | 10 | Once a day |
| G8 | Mal e | 4 | Example 4 | Y | Eye drops | 100 | 10 | Once a day |
| G9 | Mal e | 4 | Example 5 | Y | Eye drops | 200 | 10 | Once a day |

For intravitreal injection, animals were anesthetized, and a syringe with a 31-gauge needle was used to perform intravitreal injections into the right eye of the respective animals. The test material was administered as a single dose on the day of administration. The injection volume was either 1 µL/eye or 5 µL/eye.

For ocular administration, after the animals were restrained, 10 µL of the test material was instilled onto the central cornea of the right eye of the animal using a pipette, and the test material was administered once daily from the start of administration (Day 0) until the 20th day after the start of administration. The injection volume of the test material was 10 µL/eye.

### 1-3. Fundus imaging and retinal fluorescence intensity analysis

On 21st day after the start of test material administration, a mydriatic agent (Mydriacyl 1% eye drops) was instilled into the rat's right eye, and anesthesia was administered. Thereafter, a 2% fluorescein sodium salt solution was injected through the caudal vein, and images were then photographed within 2 minutes using a fundus camera (TRC-50IX, TOPCON, Japan). Retinal examination and efficacy were assessed using retinal fluorescent fundus photographs.

Image analysis was performed using ImageJ software (NIH, Bethesda, MD) to measure the fluorescein intensity in non-vascular regions of the retina. The relative levels (%) of the measured values for each individual were analyzed, with the average value of the normal control group (G1) as the reference (100%), and comparisons were made between the groups.

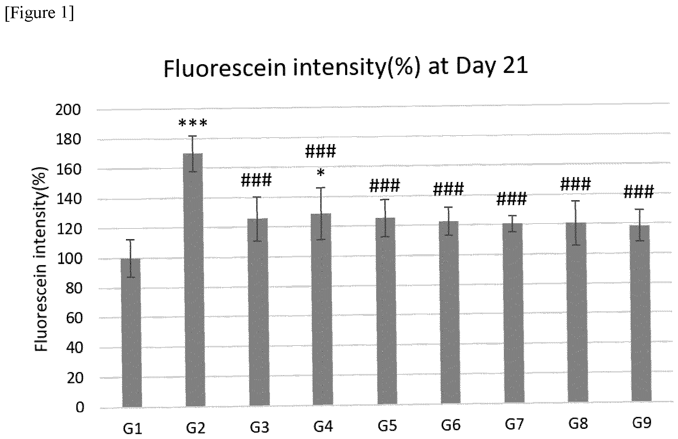

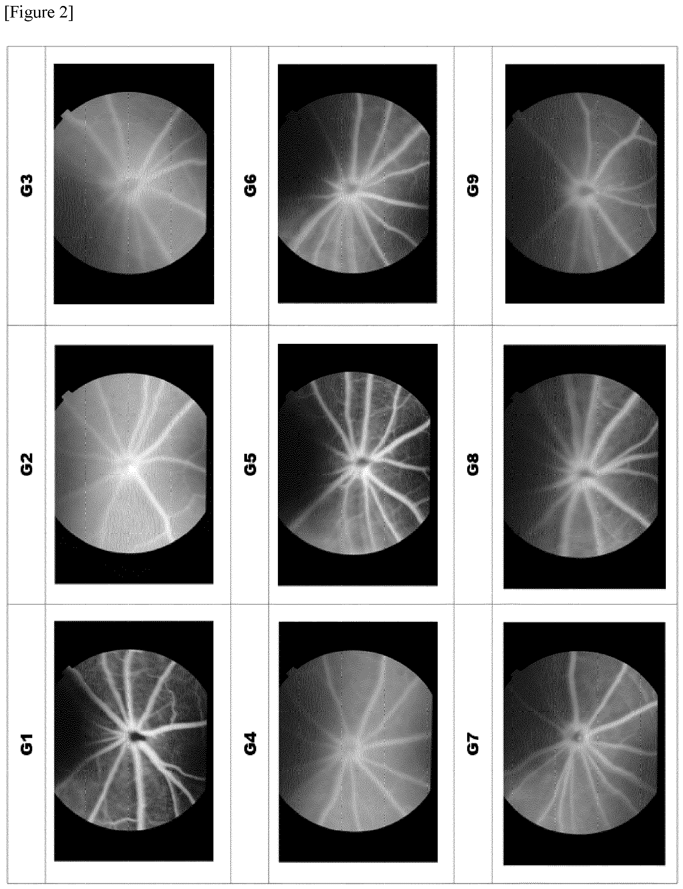

As a result, as shown in Table 2 and Figures 1 to 13, the fluorescein intensity levels of all diabetic-induced groups (G2 to G9) on the 21st day after the start of test material administration were significantly higher compared to the normal control group (G1). Particularly, the group G2, which received only the vehicle, exhibited notably high fluorescein intensity.

In the groups administered with SGLT-2 inhibitors (G5 to G9), the fluorescence intensity significantly decreased compared to G2, and the fluorescence intensity was reduced compared to the positive control groups G3 and G4 to which ranibizumab (LUCENTIS^{®}) was administered.

**[Table 2]**

| Group | Sex | Administration material | Induction | Route of administration | Fluorescence intensity on 21st day |
|---|---|---|---|---|---|
| G1 | Male | saline | N | - | 100.000 ± 12.638 |
| G2 | Male | Comparative Example 1 | Y | Eye drops | 169.756 ± 12.195*** |
| G3 | Male | Comparative Example 2 | Y | Intravitreal | 125.586 ± 14.799^{###} |
| G4 | Male | Comparative Example 2 | Y | Intravitreal | 128.359 ± 17.464*^{, ###} |
| G5 | Male | Example 1 | Y | Eye drops | 124.988 ± 12.527^{###} |
| G6 | Male | Example 2 | Y | Eye drops | 122.514 ± 9.687^{###} |
| G7 | Male | Example 3 | Y | Eye drops | 120.290 ± 5.335^{###} |
| G8 | Male | Example 4 | Y | Eye drops | 120.361 ± 14.839^{###} |
| G9 | Male | Example 5 | Y | Eye drops | 118.367 ± 10.426^{###} |

| | | | | | |
|---|---|---|---|---|---|
| ***/* A significant difference at p < 0.001/p < 0.05 compared to G1 ### A significant difference at p < 0.001 compared to G2 | | | | | |

The present invention has newly discovered SGLT-2 inhibitors that can be used for the prevention and treatment of diabetic eye diseases, confirming that SGLT-2 inhibitors can exhibit superior effects compared to the anti-VEGF monoclonal antibody therapy, such as ranibizumab (LUCENTIS^{®}), which is conventionally used for diabetic retinopathy, diabetic macular edema, and the like.

Unlike conventional therapeutic agents for eye diseases that are administered by intravitreal injection, the SGLT-2 inhibitors of the present invention (i.e., ertugliflozin, ipragliflozin, dapagliflozin, empagliflozin, and tofogliflozin) may be conveniently administered to the eyeballs as eye drops, thereby greatly improving the convenience in taking medication, and preventing damage to ocular tissues and cells caused by injections.

### 1-4. Statistical analysis

Data was assumed to be normal for the results of this experiment, and tested using a parametric one-way ANOVA. When the ANOVA results were considered to be significant, a post-hoc test was performed using a Dunnett's multiple comparison test.

Statistical analysis was performed using Prism 7.04 (GraphPad Software Inc., San Diego, CA, USA), and when the p value was less than 0.05, it was judged to be statistically significant.

The foregoing description of the present invention is intended for illustration, and it will be understood by those skilled in the art to which the invention pertains that the invention can be easily modified into other specific forms without changing the technical spirit or essential features described in the present invention. Therefore, it should be understood that the embodiments described above are only exemplary in all aspects and not limiting. For example, each of the components described as being one combined entity may be implemented separately, and similarly, components described as being separate entities may be implemented in a combined form.

It should be understood that the scope of the specification is defined by the following claims and that all changes or modifications derived from the meaning and scope of the claims and their equivalents are included in the scope of the present invention.

## Claims

1. A pharmaceutical composition for preventing or treating a diabetic eye disease, comprising a sodium-glucose cotransporter-2 (SGLT-2) inhibitor.

2. The pharmaceutical composition of claim 1, wherein the SGLT-2 inhibitor is at least one selected from the group consisting of ertugliflozin, ipragliflozin, dapagliflozin, empagliflozin, and tofogliflozin.

3. The pharmaceutical composition of claim 1, wherein the SGLT-2 inhibitor is at least one selected from the group consisting of the following Formulas 1 to 5.

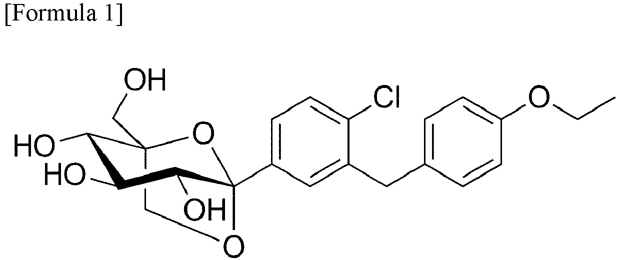

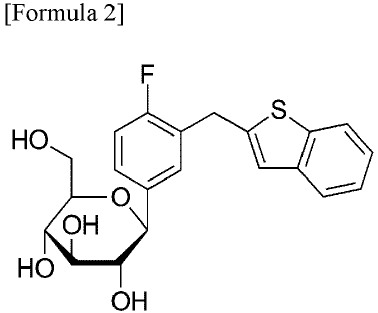

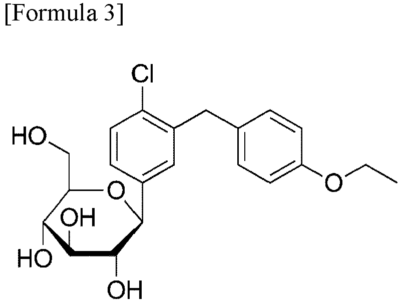

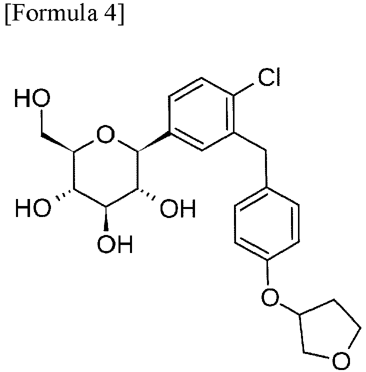

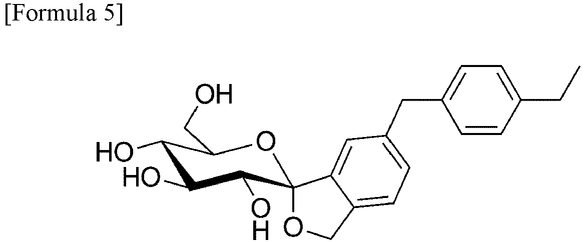

4. The pharmaceutical composition of claim 1, wherein the diabetic eye disease comprises one or more selected from the group consisting of diabetic retinopathy, macular edema, cataracts, glaucoma, and paralytic strabismus.

5. The pharmaceutical composition of claim 1, wherein the composition is for eye drop administration.

6. A method of preventing or treating a diabetic eye disease,
comprising administering the composition of claim 1 to a subject.

7. The method of claim 6, wherein the administering is administration of eye drops.

8. A use of a sodium-glucose cotransporter-2 (SGLT-2) inhibitor for the prevention or treatment of a diabetic eye disease.

9. The use of claim 8, wherein the SGLT-2 inhibitor is at least one selected from from the group consisting of ertugliflozin, ipragliflozin, dapagliflozin, empagliflozin, and tofogliflozin.
